# EUROPEAN PATENT APPLICATION

(11) **EP 3 971 175 A1**
(43) Date of publication of application: **23.03.2022**
(21) Application number: 20806591.2
(22) Date of filing: 13.05.2020
(51) Int. Cl.: C07D 401/04, A61K 31/517, A61P 35/00

(54) **CRYSTAL FORM OF QUINAZOLINONE COMPOUND AND PREPARATION METHOD THEREFOR**

(30) Priority: 13.05.2019 CN 201910394653; 21.05.2019 CN 201910423711
(71) Applicant: Luoxin Healthcare Science and Technology Development (Beijing) Ltd., Beijing 100055 (CN)
(72) Inventor: HUANG, Jingjie, Shanghai 200131 (CN); YIN, Yijie, Shanghai 200131 (CN); YAO, Ting, Shanghai 200131 (CN); YU, Tao, Shanghai 200131 (CN); WU, Chengde, Shanghai 200131 (CN); DONG, Jiaqiang, Shanghai 201210 (CN); SHI, Bin, Shanghai 201210 (CN); TANG, Wei, Shanghai 201210 (CN); YANG, Wenqian, Shanghai 201210 (CN); WANG, Tie-Lin, Shanghai 201210 (CN)
(74) Representative: Haseltine Lake Kempner LLP
(86) International application number: PCT/CN2020/089972
(87) International publication number: WO 2020/228729

(57) **Abstract**

The present invention relates to crystal forms of a compound of formula (I), which act as a PI3Kα inhibitor, and preparation methods thereof, and the use thereof in the preparation of a medicine for treating solid tumors.

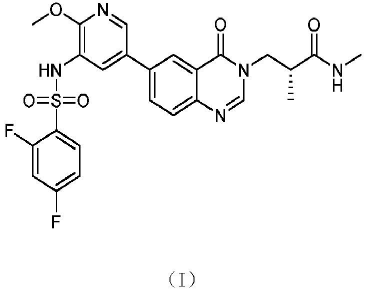

## Description

The present application claims the priorities of Chinese patent application CN201910394653.5 filed on May 13, 2019 and Chinese patent application CN201910423711.2 filed on May 21, 2019. The contents of the Chinese patent application are incorporated herein by reference in their entireties.

### Technical Field

The present disclosure relates to crystal forms of a compound as a PI3Kα inhibitor and preparation methods thereof, and relates to a use thereof in the preparation of a medicament for treating solid tumors.

### Background

Phosphatidylinositol-3-kinase (PI3K) is a lipid kinase composed of a regulatory subunit p85 or p101, and a catalytic subunit p110 (further divided into four subtypes: p110α, p110β, p110δ, p110γ), which activates downstream Akt etc by catalyzing the phosphorylation of the inositol ring 3'-OH group in phosphatidylinositol 4,5-bisphosphate (PIP2) to phosphatidylinositol 3,4,5-triphosphate (PIP3), so it plays a key role in cell proliferation, survival and metabolism. PI3K is overexpressed in tumor cells, resulting in rapid proliferation and growth of tumor cells.

The tumor suppressor gene PTEN (Phosphatase and TENsin homolog deleted on chromosome 10) dephosphorylates PIP3 to generate PIP2, resulting in negative feedback regulation of the PI3K signaling pathway, inhibiting cell proliferation and promoting apoptosis. Frequent occurrence of PI3K gene mutations and amplifications in cancer and PTEN gene deletion in cancer suggest that PI3K overexpression is closely related to tumorigenesis.

Zhang hao et al. (Bioorganic Medicinal Chemistry, 2015 (23): 7765-7776.) found that compounds A2 and A10 (control examples R011 and R012) have a good inhibitory effect on PI3K. BYL-719(WO2010/029082), a PI3Kα selective inhibitor developed by Novartis, is currently in the pre-registration stage, and is the compound with the highest research status of similar target inhibitors in the world.

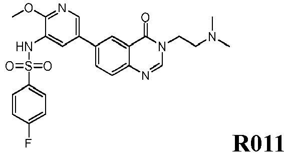

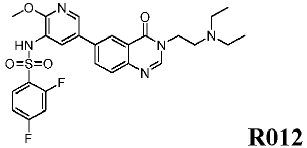

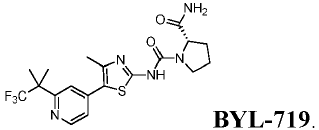

### Content of the present invention

The present disclosure provides a crystal form A of a compound represented by formula (I), wherein the X-ray powder diffraction pattern thereof has characteristic diffraction peaks at the following angles of 2θ: 4.8 ± 0.20°, 12.6 ± 0.20°, and 17.3 ± 0.20°.

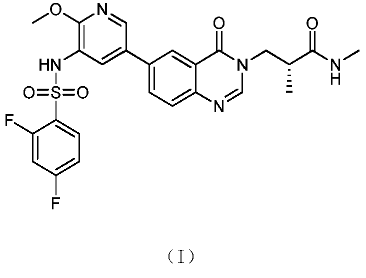

In some embodiments of the present disclosure, the X-ray powder diffraction pattern of the crystal form A has characteristic diffraction peaks at the following angles of 2θ: 4.8±0.2°, 5.7±0.2°, 6.3±0.2°, 11.5±0.2°, 12.6±0.2°, 13.5±0.2°, 17.3±0.2° and 21.5±0.2°.

In some embodiments of the present disclosure, the X-ray powder diffraction pattern of the crystal form A has characteristic diffraction peaks at the following angles of 2θ: 4.8 ± 0.2°, 5.7 ± 0.2°, 6.3 ± 0.2°, 10.1 ± 0.2°, 11.5 ± 0.2°, 12.6 ± 0.2°, 13.5 ± 0.2°, 15.8 ± 0.2°, 17.3 ± 0.2°, 19.2 ± 0.2°, and 21.5 ± 0.2°.

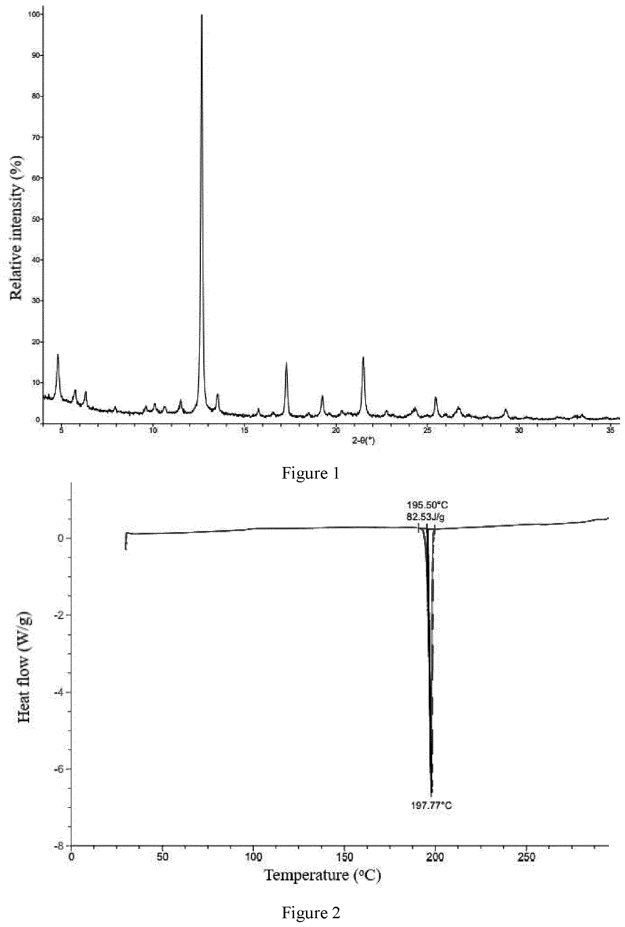

In some embodiments of the present disclosure, the X-ray powder diffraction pattern of the crystal form A is shown in Figure 1.

In some embodiments of the present disclosure, the analytical data of the X-ray powder diffraction pattern of the crystal form A is shown in Table 1.

**Table 1: Analytical data of the X-ray powder diffraction pattern of the crystal form A of the compound represented by formula (I)**

| No. | Angles of 2θ (°) | d-Spacing (Å) | Relative intensity (%) | No. | Angles of 2θ (°) | d-Spacing (Å) | Relative intensity (%) |
|---|---|---|---|---|---|---|---|
| 1 | 4.8 | 18.4 | 11.5 | 15 | 20.3 | 4.4 | 1.4 |
| 2 | 5.7 | 15.4 | 3.3 | 16 | 20.6 | 4.3 | 0.7 |
| 3 | 6.3 | 14.0 | 3.8 | 17 | 21.5 | 4.1 | 14.6 |
| 4 | 8.0 | 11.1 | 1.3 | 18 | 22.8 | 3.9 | 1.6 |
| 5 | 9.6 | 9.2 | 1.7 | 19 | 23.1 | 3.8 | 0.7 |
| 6 | 10.1 | 8.8 | 2.5 | 20 | 24.0 | 3.7 | 0.9 |
| 7 | 10.6 | 8.3 | 1.5 | 21 | 24.3 | 3.6 | 2.5 |
| 8 | 11.5 | 7.7 | 3.3 | 22 | 25.4 | 3.5 | 5 |
| 9 | 12.6 | 7.0 | 100 | 23 | 26.7 | 3.3 | 2.7 |
| 10 | 13.5 | 6.5 | 4.3 | 24 | 27.2 | 3.3 | 0.8 |
| 11 | 15.8 | 5.6 | 2 | 25 | 29.2 | 3.0 | 2.3 |
| 12 | 17.3 | 5.1 | 13.4 | 26 | 32.1 | 2.8 | 0.6 |
| 13 | 19.2 | 4.6 | 5.3 | 27 | 33.1 | 2.7 | 0.9 |
| 14 | 19.7 | 4.5 | 0.8 | 28 | 33.4 | 2.7 | 1.2 |

In some embodiments of the present disclosure, the differential scanning calorimetry curve of the crystal form A has an endothermic peak with onset at 195.5±3.0 °C.

In some embodiments of the present disclosure, the DSC pattern of the crystal form A is shown in Figure 2.

In some embodiments of the present disclosure, the thermogravimetric analysis curve of the crystal form A has a weight loss of 0.16% occurred at 151.6±3.0 °C.

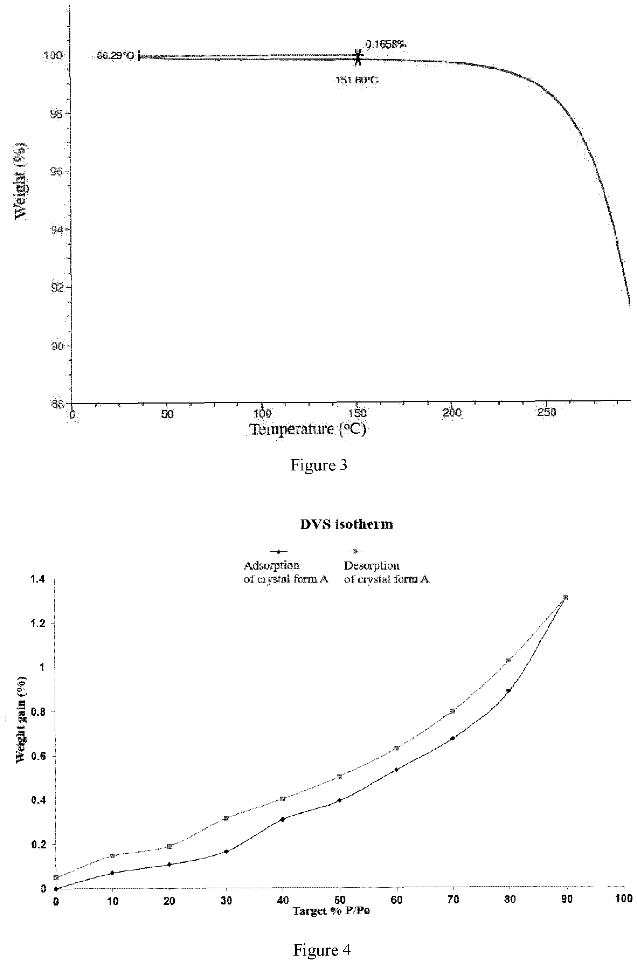

In some embodiments of the present disclosure, the TGA pattern of the crystal form A is shown in Figure 3.

The present disclosure also provides a crystal form B of the compound represented by formula (I), wherein the X-ray powder diffraction pattern thereof has characteristic diffraction peaks at the following angles of 2θ: 5.0 ± 0.2°, 9.9 ± 0.2°, and 12.3 ± 0.2°.

In some embodiments of the present disclosure, the X-ray powder diffraction pattern of the crystal form B has characteristic diffraction peaks at the following angles of 2θ: 5.0 ± 0.2°, 9.9 ± 0.2°, 12.3 ± 0.2°, 14.9 ± 0.2°, 20.2 ± 0.2°, 24.4 ± 0.2°, 27.1 ± 0.2°, and 30.1 ± 0.2°.

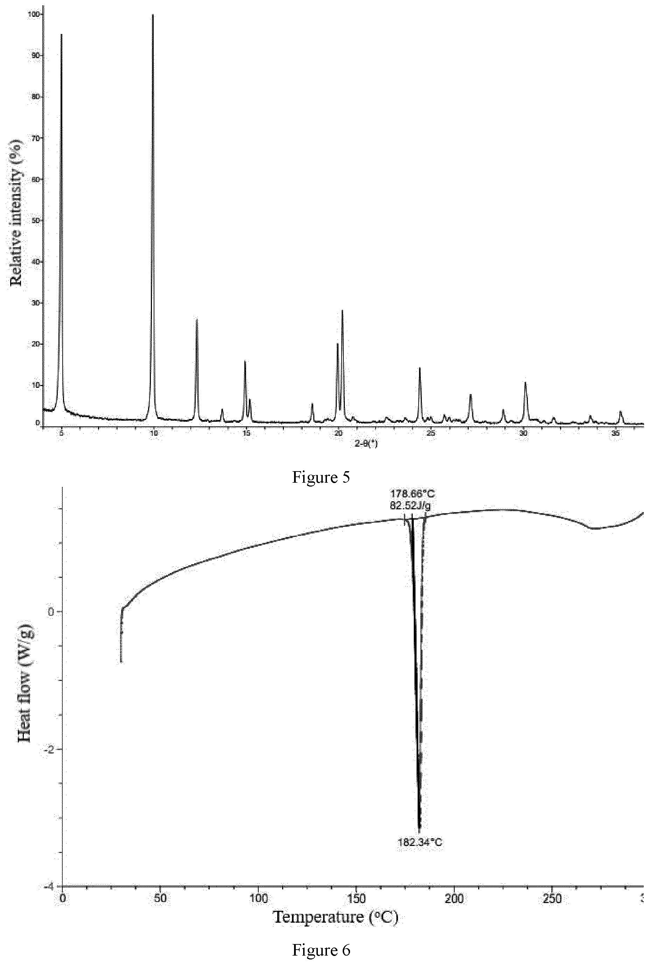

In some embodiments of the present disclosure, the X-ray powder diffraction pattern of the crystal form B is shown in Figure 5.

In some embodiments of the present disclosure, the analytical data of the X-ray powder diffraction pattern of the crystal form B is shown in Table 2.

**Table 2: Analytical data of the X-ray powder diffraction pattern of the crystal form B of the compound represented by formula (I)**

| No. | Angles of 2θ (°) | d-Spacing (Å) | Relative intensity (%) | No. | Angles of 2θ (°) | d-Spacing (Å) | Relative intensity (%) |
|---|---|---|---|---|---|---|---|
| 1 | 5.0 | 17.7 | 93.3 | 15 | 24.8 | 3.6 | 1.2 |
| 2 | 9.9 | 8.9 | 100 | 16 | 25.0 | 3.6 | 1.3 |
| 3 | 12.3 | 7.2 | 25.1 | 17 | 25.7 | 3.5 | 2 |
| 4 | 13.7 | 6.5 | 3.1 | 18 | 26.0 | 3.4 | 1.3 |
| 5 | 14.9 | 5.9 | 14.9 | 19 | 26.3 | 3.4 | 0.6 |
| 6 | 15.2 | 5.8 | 5.5 | 20 | 26.5 | 3.4 | 0.6 |
| 7 | 18.6 | 4.8 | 4.6 | 21 | 27.1 | 3.3 | 6.9 |
| 8 | 19.4 | 4.6 | 0.7 | 22 | 28.9 | 3.1 | 3.2 |
| 9 | 19.9 | 4.4 | 19.1 | 23 | 29.4 | 3.0 | 0.5 |
| 10 | 20.2 | 4.4 | 27.5 | 24 | 30.1 | 3.0 | 9.9 |
| 11 | 22.6 | 3.9 | 1.2 | 25 | 30.7 | 2.9 | 0.9 |
| 12 | 23.2 | 3.8 | 0.4 | 26 | 31.6 | 2.8 | 1.4 |
| 13 | 23.6 | 3.8 | 1 | 27 | 33.6 | 2.7 | 1.9 |
| 14 | 24.4 | 3.6 | 13.3 | 28 | 33.9 | 2.6 | 0.6 |

In some embodiments of the present disclosure, the differential scanning calorimetry curve of the crystal form B has an endothermic peak with onset at 178.7±3.0 °C.

In some embodiments of the present disclosure, the DSC pattern of the crystal form B is shown in Figure 6.

In some embodiments of the present disclosure, the thermogravimetric analysis curve of the crystal form B has a weight loss of 1.03% occurred at 63.4±3.0 °C.

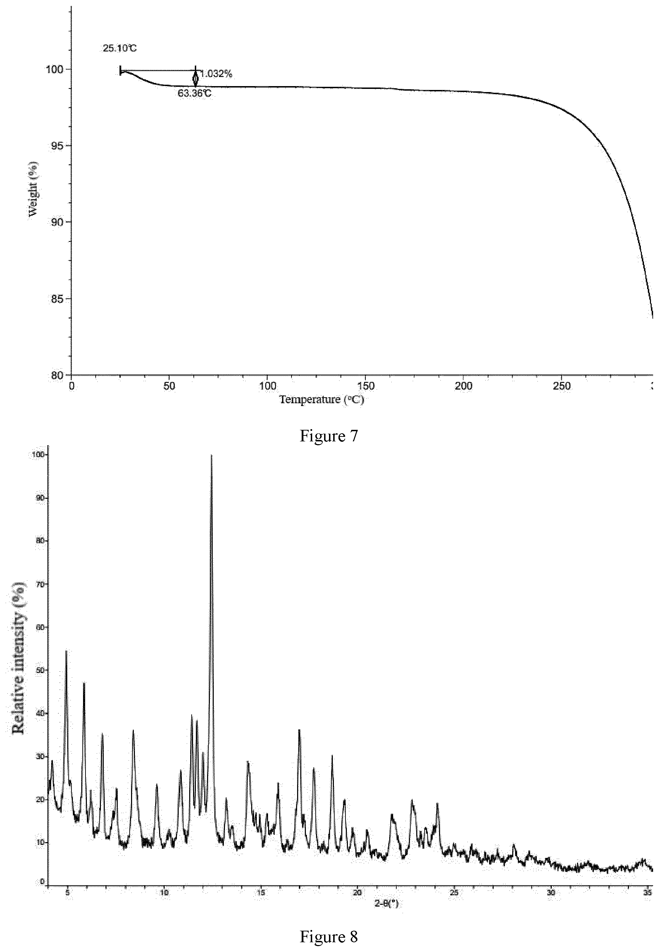

In some embodiments of the present disclosure, the TGA pattern of the crystal form B is shown in Figure 7.

The present disclosure provides a crystal form C of the compound represented by formula (I), wherein the X-ray powder diffraction pattern thereof has characteristic diffraction peaks at the following angles of 2θ: 4.9±0.2°, 5.8±0.2°, 6.8±0.2°, 8.4±0.2° and 12.4±0.2°.

In some embodiments of the present disclosure, the X-ray powder diffraction pattern of the crystal form C has characteristic diffraction peaks at the following angles of 2θ: 4.9 ± 0.2°, 5.8 ± 0.2°, 6.8 ± 0.2°, 8.4 ± 0.2°, 10.8 ± 0.2°, 11.7 ± 0.2°, 12.4 ± 0.2°, 14.3 ± 0.2°, 17.0 ± 0.2°, 17.7 ± 0.2°, and 18.7 ± 0.2°.

In some embodiments of the present disclosure, the X-ray powder diffraction pattern of the crystal form C is shown in Figure 8.

In some embodiments of the present disclosure, the analytical data of the X-ray powder diffraction pattern of the crystal form C is shown in Table 3.

**Table 3: Analytical data of the X-ray powder diffraction pattern of the crystal form C of the compound represented by formula (I)**

| No. | Angles of 2θ (°) | d-Spacing (Å) | Relative intensity (%) | No. | Angles of 2θ (°) | d-Spacing (Å) | Relative intensity (%) |
|---|---|---|---|---|---|---|---|
| 1 | 4.2 | 20.9 | 9.8 | 19 | 14.3 | 6.2 | 22.7 |
| 2 | 4.9 | 17.9 | 43.5 | 20 | 14.7 | 6.0 | 8.3 |
| 3 | 5.1 | 17.2 | 8.8 | 21 | 14.9 | 5.9 | 7.7 |
| 4 | 5.8 | 15.1 | 38.2 | 22 | 15.3 | 5.8 | 8.1 |
| 5 | 6.2 | 14.2 | 9.3 | 23 | 15.9 | 5.6 | 17.3 |
| 6 | 6.8 | 13.0 | 27.6 | 24 | 17.0 | 5.2 | 31.9 |
| 7 | 7.4 | 12.0 | 8.1 | 25 | 17.2 | 5.1 | 9.5 |
| 8 | 7.5 | 11.7 | 14.7 | 26 | 17.7 | 5.0 | 21.3 |
| 9 | 8.4 | 10.5 | 31.4 | 27 | 18.7 | 4.7 | 25.4 |
| 10 | 9.6 | 9.2 | 16.4 | 28 | 19.3 | 4.6 | 14.4 |
| 11 | 10.3 | 8.6 | 4.3 | 29 | 19.7 | 4.5 | 6.2 |
| 12 | 10.8 | 8.2 | 19.3 | 30 | 20.5 | 4.3 | 7.2 |
| 13 | 11.4 | 7.7 | 28.4 | 31 | 21.8 | 4.1 | 12 |
| 14 | 11.7 | 7.6 | 31.6 | 32 | 22.8 | 3.9 | 14.1 |
| 15 | 12.0 | 7.4 | 23.4 | 33 | 23.5 | 3.8 | 7.4 |
| 16 | 12.4 | 7.1 | 100 | 34 | 23.9 | 3.7 | 6.7 |
| 17 | 13.2 | 6.7 | 12.3 | 35 | 24.1 | 3.7 | 13 |
| 18 | 13.5 | 6.5 | 5.4 | 36 | 25.0 | 3.6 | 3.6 |

The present disclosure also provides a crystal form D of the compound represented by formula (I), wherein the X-ray powder diffraction pattern thereof has characteristic diffraction peaks at the following angles of 2θ: 5.1 ± 0.2°, 7.8 ± 0.2°, and 11.8 ± 0.2°.

In some embodiments of the present disclosure, the X-ray powder diffraction pattern of the crystal form D has characteristic diffraction peaks at the following angles of 2θ: 5.1 ± 0.2°, 6.5 ± 0.2°, 7.8 ± 0.2°, 11.8 ± 0.2°, 15.4 ± 0.2°, 16.5 ± 0.2°, 17.4 ± 0.2°, and 23.8 ± 0.2°.

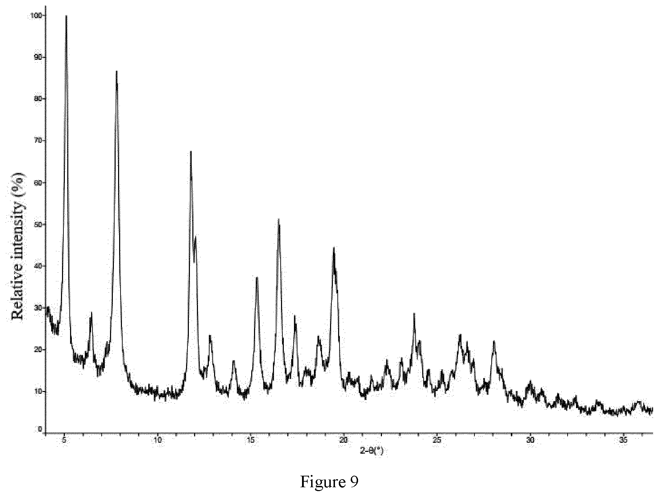

In some embodiments of the present disclosure, the X-ray powder diffraction pattern of the crystal form D is shown in Figure 9.

In some embodiments of the present disclosure, the analytical data of the X-ray powder diffraction pattern of the crystal form D is shown in Table 4.

**Table 4: Analytical data of the X-ray powder diffraction pattern of the crystal form D of the compound represented by formula (I)**

| No. | Angles of 2θ (°) | d-Spacing (Å) | Relative intensity (%) | No. | 2θ (°) | d-Spacing (Å) | Relative intensity (%) |
|---|---|---|---|---|---|---|---|
| 1 | 5.1 | 17.2 | 100 | 18 | 22.3 | 4.0 | 8.7 |
| 2 | 6.5 | 13.7 | 16.3 | 19 | 23.8 | 3.7 | 23.1 |
| 3 | 7.3 | 12.1 | 8.3 | 20 | 24.0 | 3.7 | 13.1 |
| 4 | 7.8 | 11.3 | 95.4 | 21 | 24.6 | 3.6 | 4.8 |
| 5 | 11.8 | 7.5 | 71.9 | 22 | 25.3 | 3.5 | 5.9 |
| 6 | 12.0 | 7.3 | 44.3 | 23 | 26.3 | 3.4 | 16.7 |
| 7 | 12.8 | 6.9 | 13.5 | 24 | 26.6 | 3.3 | 14.3 |
| 8 | 14.1 | 6.3 | 10.1 | 25 | 27.0 | 3.3 | 8.4 |
| 9 | 15.4 | 5.8 | 35.5 | 26 | 28.1 | 3.2 | 16.1 |
| 10 | 16.5 | 5.4 | 50.3 | 27 | 28.5 | 3.1 | 8.1 |
| 11 | 17.4 | 5.1 | 19.8 | 28 | 30.0 | 3.0 | 7.3 |
| 12 | 17.9 | 4.9 | 4.4 | 29 | 30.6 | 2.9 | 3.7 |
| 13 | 18.6 | 4.8 | 11 | 30 | 31.5 | 2.8 | 4 |
| 14 | 19.5 | 4.6 | 41 | 31 | 32.4 | 2.8 | 4.2 |
| 15 | 20.2 | 4.4 | 4.4 | 32 | 33.6 | 2.7 | 3.4 |
| 16 | 20.8 | 4.3 | 4.4 | 33 | 35.8 | 2.5 | 3.1 |
| 17 | 21.4 | 4.1 | 4.5 | 34 | 38.0 | 2.4 | 3.5 |

The present disclosure provides a preparation method of the crystal form A of the compound represented by formula (I), comprising:
(1) adding the compound represented by formula (I) into a solvent to make it into a suspension or solution;
(2) putting the suspension or solution in a constant temperature mixer, shaking, then centrifuging, and drying to obtain the crystal form A of the compound represented by formula (I).

In some embodiments of the present disclosure, the above preparation method, wherein the solvent is selected from alcohol solvent and ester solvent.

In some embodiments of the present disclosure, the above preparation method, wherein the solvent is selected from ethanol, *n*-butanol, *tert*-butanol, isopropanol, ethyl formate and ethyl acetate.

The present disclosure provides a preparation method of the crystal form B of the compound represented by formula (I), comprising:

(1) adding the compound represented by formula (I) into a solvent to make it into a suspension or solution;

(2) putting the suspension or solution in a constant temperature mixer, shaking, then centrifuging, and drying to obtain the crystal form B of the compound represented by formula (I).

The present disclosure provides a preparation method of the crystal form A of the compound represented by formula (I), comprising:
(1) adding the compound represented by formula (I) into a solvent and heating to dissolve;
(2) cooling the solution until solid precipitates, stirring and filtering to obtain the crystal form A of the compound represented by formula (I).

In some embodiments of the present disclosure, the above preparation method, wherein, the solvent is selected from methanol-water, ethanol-water and acetone-water.

In some embodiments of the present disclosure, the above preparation method, wherein, the solvent is selected from methanol-water (2:1), ethanol-water (2:1), acetone-water (2:1), ethanol-water (1:3).

In some embodiments of the present disclosure, the above preparation method, wherein, the stirring temperature is 25°C to 60°C.

In some embodiments of the present disclosure, the above preparation method, wherein, the stirring time is 12 hours to 24 hours.

In some embodiments of the present disclosure, the above preparation method, wherein, the weight-volume ratio of the compound to the solvent is 1 g: 7 to 10 mL.

The present disclosure also provides a use of the above crystal form in the manufacture of a medicament for treating PI3Kα inhibitor-related diseases.

In some embodiments of the present disclosure, the above use, wherein, PI3Kα inhibitor-related medicaments are medicaments for tumors.

The present disclosure also provides a pharmaceutical composition comprising the crystal form and a pharmaceutically acceptable excipient. The crystal form may be a therapeutically effective amount.

The present disclosure also provides a use of the crystal form or the pharmaceutical composition in the preparation of PI3K inhibitors. The PI3K inhibitor may be an inhibitor of one or more of PI3Kα, PI3Kβ, PI3Kδ and PI3Kγ, preferably an inhibitor of PI3Kα.

The present disclosure also provides a use of the crystal form or the pharmaceutical composition in the preparation of a medicament. The medicament may be a medicament for treating tumors or a medicament for PI3K-related diseases. The PI3K-related disease may be a tumor. The PI3K may be one or more of PI3Kα, PI3Kβ, PI3Kδ and PI3Kγ, preferably PI3Kα.

The present disclosure also provides a method for treating tumor or PI3K-related diseases comprising administering a therapeutically effective amount of the crystal form or the pharmaceutical composition to a patient. The PI3K-related disease may be a tumor. The PI3K may be one or more of PI3Kα, PI3Kβ, PI3Kδ and PI3Kγ, preferably PI3Kα.

In the present disclosure, the tumor may be one or more of breast cancer, ovarian cancer, head and neck cancer, esophageal cancer, lung cancer, cervical cancer, neuroendocrine prostate cancer, endometrial cancer, bladder cancer and colorectal cancer, preferably breast cancer and/or ovarian cancer.

### Definition and description

Unless otherwise indicated, the following terms and phrases used in this document are intended to have the following meanings. A specific term or phrase should not be considered indefinite or unclear in the absence of a particular definition, but should be understood in the ordinary sense. When a trade name appears herein, it is intended to refer to its corresponding commodity or active ingredient thereof.

The intermediate compounds of the present disclosure can be prepared by various synthetic methods known to those skilled in the art, including the embodiments described below, the embodiments formed by combining the embodiments described below with other chemical synthesis methods, and equivalent alternatives well-known to those skilled in the art. Preferred embodiments include, but are not limited to, the embodiments of the present disclosure.

The chemical reactions of the embodiments of the present disclosure are carried out in a suitable solvent, and the solvent should be suitable for the chemical change, and the reagents and materials required therefor of the present disclosure. In order to obtain the compounds of the present disclosure, it is sometimes necessary for those skilled in the art to modify or select the synthetic steps or reaction schemes based on the existing embodiments.

The present disclosure will be specifically described below by way of embodiments, but the scope of the present disclosure is not limited thereto.

All solvents used in the present disclosure are commercially available and can be directly used without further purification.

The solvents used in the present disclosure are commercially available. The present disclosure adopts the following abbreviations: DCM stands for dichloromethane; DMF stands for *N*,*N*-dimethylformamide; DMSO stands for dimethyl sulfoxide; EtOH stands for ethanol; MeOH stands for methanol; TFA stands for trifluoroacetic acid; ATP stands for adenosine triphosphate; HEPES stands for 4-hydroxyethyl piperazine ethanesulfonic acid; MgCl₂ stands for magnesium dichloride.

### Technical effect

The compounds of the present disclosure have good crystal stability and are easy to be made into drugs. The compound of the present disclosure has a good inhibitory activity on PI3K kinase, and at the same time, it has a high subtype selectivity for PI3Kβ/γ/δ; it can also well inhibit the phosphorylation level of Akt which is the downstream of PI3K in cells, and also exhibits high subtype selectivity. The compound of the present disclosure can obviously inhibit the growth of tumors *in vivo,* and also shows an obvious time-dependent and dose-dependent inhibitory effect on the phosphorylation level of Akt which is the downstream of PI3K in animals. The compound of the present disclosure has no significant inhibitory effect on hERG and CYP enzymes, and is metabolically stable in liver cells of humans, rats, mice, dogs and monkeys.

### 1.1 X-ray powder diffractometer (XRPD)

Instrument model: Bruker D8 advance X-ray diffractometer
Detection method: about 10-20 mg of the sample was used for XRPD detection.
Detailed XRPD parameters were as follows:
   X-ray generator: Cu, kα, (λ=1.54056Ǻ).
   Tube voltage: 40 kV, tube current: 40 mA.
   Emission slit: 0.60 mm
   Detector slit: 10.50 mm
   Anti-scattering slit: 7.10 mm
   Scanning range(angle of 2θ): 4-40 deg.
   Step size: 0.02 deg
   Rate: 0.1 second
   Rotation speed of sample tray: 15 rpm

### 1.2 Differential Scanning Calorimeter (DSC)

Instrument model: DSC Q2000 differential scanning calorimeter
Detection method: 0.5-1mg of the sample was placed in a DSC aluminum crucible for testing, under the condition of 50 mL/min N₂ at a heating rate of 10 °C/min, the sample was heated from room temperature to 300 °C.

### 1.3 Thermal Gravimetric Analyzer (TGA)

Instrument Model: TA Q5000 IR thermal gravimetric analyzer
Detection method: 2-5 mg of the sample was placed in a TGA platinum crucible for testing, under the condition of 25 mL/min N₂ at a heating rate of 10 °C/min, the sample was heated from room temperature to 300 °C, or until a weight loss of 20%.

### 1.4 Dynamic Vapor Sorption (DVS) method of the present disclosure

Instrument model: SMS DVS Advantage dynamic vapor sorption instrument
Detection conditions: 10-20 mg of sample was placed in a DVS sample tray for testing.

Detailed DVS parameters were as follows:
Temperature: 25 °C
Equilibrium: dm/dt=0.01 %/min: (shortest: 10 min, longest: 180 min)
Drying: drying at 0% RH for 120 min
RH (%) test gradient: 10%
RH (%) test gradient range: 0%-90%-0%

Hygroscopicity evaluation is classified as follows:

| **Classification for hygroscopicity** | ΔW% |
|---|---|
| Deliquescence | Absorbing sufficient water to form liquid |
| Highly hygroscopic | ΔW% ≥ 15% |
| Hygroscopic | 15% ≥ ΔW% ≥ 2% |
| slightly hygroscopic | 2% > ΔW% ≥ 0.2% |
| non-hygroscopic or almost non-hygroscopic | ΔW% < 0.2% |

| | |
|---|---|
| [0096] Note: ΔW% indicates the moisture-absorbing weight gain of the sample at 25±1 °C and 80±2% RH | |

### 1.5 High Performance Liquid Chromatograph Method

Sample concentration: 0.5 mg/mL

The chromatographic conditions of the HPLC method for solid stability test were shown in Table 5 below:

**Table 5**

| | | | |
|---|---|---|---|
| Chromatographic column | Waters Xbridge Sheild RP18 3.5µm. 4.6^{∗}150mm PN: 186003045 | | |
| Wavelength | 210nm | | |
| Column temperature | 35 °C | | |
| Flow rate | 0.8 mL/min | | |
| Injection volume | 10.0µL | | |
| Mobile phase | Mobile phase A: water (0.04% TFA) Mobile phase B: 100% acetonitrile | | |
| Gradient program | Time/minute | A% | B% |
| | 0.01 | 95 | 5 |
| | 5.00 | 95 | 5 |
| | 10.00 | 75 | 25 |
| | 18.00 | 75 | 25 |
| | 45.00 | 25 | 75 |
| | 50.00 | 5 | 95 |
| | 51.00 | 95 | 5 |
| | 56.00 | 95 | 5 |
| | 56.01 | Stop | |
| Data collection time | 56.01min | | |
| Diluent | Acetonitrile: water =1:1(v/v) | | |
| Needle washing liquid | Acetonitrile: water =1:1(v/v) | | |

### Brief description of the drawings

Figure 1 is the X-ray powder diffraction pattern of the crystal form A of the compound represented by formula (I) measured by Cu-Ka radiation.
Figure 2 is the DSC thermogram of the crystal form A of the compound represented by formula (I).
Figure 3 is the TGA thermogram of the crystal form A of the compound represented by formula (I).
Figure 4 is the DVS isotherm plot of the crystal form A of the compound represented by formula (I).
Figure 5 is the X-ray powder diffraction pattern of the crystal form B of the compound represented by formula (I) measured by Cu-Ka radiation.
Figure 6 is the DSC thermogram of the crystal form B of the compound represented by formula (I).
Figure 7 is the TGA thermogram of the crystal form B of the compound represented by formula (I).
Figure 8 is the X-ray powder diffraction pattern of the crystal form C of the compound represented by formula (I) measured by Cu-Ka radiation.
Figure 9 is the X-ray powder diffraction pattern of the crystal form D of the compound represented by formula (I) measured by Cu-Ka radiation.
Figure 10 shows the protein expression of p-AKT in BT474 tumor tissues of the crystal form A of the compound represented by formula (I) 0.5h, 4h, 24h after administration;
   wherein, P-AKT stands for phosphorylated Akt protein, and β-actin stands for β-actin.
Figure 11 is the DVS isotherm plot of the crystal form B of the compound represented by formula (I).

### Detailed description of the embodiment

For a better understanding of the content of the present disclosure, the present disclosure is described in detail through the embodiments, which does not mean any limitation on the present disclosure.

### Embodiment 1: Preparation of the compound represented by formula (I)

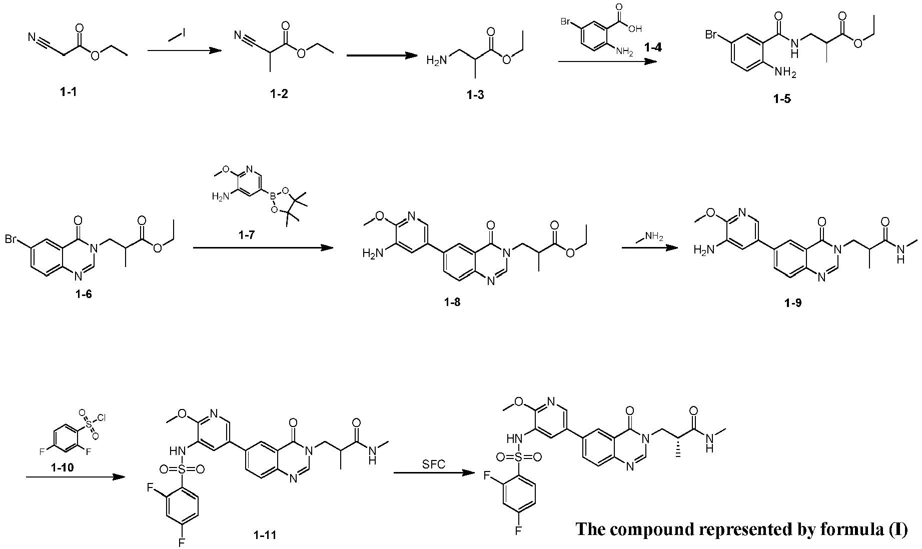

### Step1: Synthesis of compound 1-2.

Compound 1-1 (20.00g, 176.82mmol, 18.87mL), methyl iodide (37.65g, 265.23mmol, 16.51mL) and potassium carbonate (48.88g, 353.64mmol) were added to DMF(100mL), and the system was stirred at 25°C for 48 hours. After the reaction was completed, the solvent was removed under reduced pressure. Then the mixture was diluted with water (200 mL), and extracted with dichloromethane (200 mL), the organic phase was concentrated under reduced pressure, and the residue was separated by chromatography column (ethyl acetate: petroleum ether=0%-15%) to obtain compound **1-2.** ¹H NMR (400 MHz, CDCl₃) δ: 4.23 - 4.34 (m, 2 H), 3.56 (q, *J*=7.4 Hz, 1 H), 1.61 (dd, *J*=7.5, 1.5 Hz, 3 H), 1.31 - 1.37 (m, 3 H).

### Step2: Synthesis of compound 1-3.

Compound **1-2** (2.30g, 18.09mmol) was dissolved in ethanol (20.00mL), and then Raney nickel (1.55g, 18.09mmol) was added under nitrogen gas flow. The system was stirred at 25°C for 24 hours under 50 Pa hydrogen pressure. After the reaction was completed, the system was filtered, the filtrate was concentrated under reduced pressure, and the residue was separated by chromatography column (methanol: dichloromethane=0%-6%) to obtain compound **1-3.** ¹H NMR (400 MHz, DMSO-*d*₆) δ: 4.01 - 4.09 (m, 2 H), 2.72 (dd, *J*=12.5, 7.0 Hz, 1 H), 2.55 - 2.62 (m, 1 H), 2.35 - 2.45 (m, 1 H), 1.18 (t, *J*=7.3 Hz, 3 H), 1.04 (d, *J*=7.0 Hz, 3 H).

### Step 3: Synthesis of compound 1-5.

Compound **1-4** (1.20g, 5.55mmol), compound **1-3** (800mg, 6.11mmol), EDCI (1.09g, 5.66mmol), 2-hydroxypyridine-*N*-oxide (722mg, 6.49mmol), triethylamine (2.25g, 22.20mmol, 3.08mL) were added to dichloromethane (120mL), and the system was stirred at 50°C for 16 hours. After the reaction was completed, the reaction solution was diluted with water (200 mL), extracted with dichloromethane (200 mL), the organic phase was concentrated under reduced pressure, and the residue was separated by chromatography column (methanol: dichloromethane=0%-2%) to obtain compound **1-5.** ¹H NMR (400 MHz, DMSO-*d*₆) δ : 8.46 (t, *J*=5.6 Hz, 1 H), 7.61 (d, *J*=2.3 Hz, 1 H), 7.26 (dd, *J*=8.8, 2.3 Hz, 1 H), 6.67 (d, *J*=8.8 Hz, 1 H), 6.52 (br s, 2 H), 4.06 (q, *J*=7.1 Hz, 2 H), 3.37 - 3.45 (m, 1 H), 3.21 - 3.29 (m, 1 H), 2.67 - 2.80 (m, 1 H), 1.17 (t, *J*=7.2 Hz, 3 H), 1.08 (d, *J*=7.0 Hz, 3 H).

### Step 4: Synthesis of compound 1-6

Compound **1-5** (1.00g, 2.86mmol) was added to formic acid (24.40g, 530.09mmol, 20.00mL), and the system was stirred at 100°C for 16 hours. After the reaction was completed, the reaction solution was concentrated under reduced pressure, and the residue was separated by chromatography column (ethyl acetate: petroleum ether =0%-40%) to obtain compound **1-6.** MS-ESI *m*/*z:* 340.8 [M+H]⁺.

### Step 5: Synthesis of compound 1-8

Compound **1-6** (2 g, 5.90 mmol) was dissolved in dioxane (20 mL) and water (4 mL), then compound **1-7** (1.77 g, 7.08 mmol), Pd(dppf)Cl₂ (963.06 mg, 1.18 mmol) and potassium acetate (2.31 g, 23.59 mmol) were added thereto, and the reaction solution was stirred under nitrogen protection for 3 hours at 100°C. After the reaction was completed, the reaction solution was evaporated to dryness. The residue was separated by chromatography column (eluent: methanol/dichloromethane= 5-10%) to obtain the target compound **1-8.** MS-ESI *m*/*z:* 383.1 [M+H]⁺.

### Step 6: Synthesis of compound 1-9

Compound **1-8** (2.3 g, 6.01 mmol) was dissolved in methylamine ethanol solution (2 M, 50 mL), and the reaction solution was stirred at 80°C for 10 hours. After the reaction was completed, the reaction solution was evaporated to dryness. The target compound **1-9** was obtained. MS-ESI *m*/*z:* 368.1 [M+H]⁺.

### Step 7: Synthesis of compound 1-11

Compound **1-9** (0.3 g, 816.55 µmol) was dissolved in pyridine (5 mL), then compound **1-10** (157.82 mg, 742.32 µmol, 99.88 µL) was added thereto, and the reaction solution was stirred at 25°C for 10 hours. After the reaction was completed, the reaction solution was evaporated to dryness. The mixture was separated by preparative HPLC separation (TFA). The target compound **1-11** was obtained. MS-ESI *m*/*z:* 544.1 [M+H]⁺.

### Step 8: Synthesis of the compound represented by formula (I)

Compound **1-11** was separated by supercritical fluid chromatography (separation conditions: column: AD (250mm^{∗}30mm, 10µm); mobile phase: [0.1%NH₃H₂O EtOH]; B (acetonitrile)%: 55%-55%), the compound represented by formula (I) was obtained (retention time was 0.711 min), ¹H NMR (400 MHz, DMSO-*d*₆) δ = 8.22 - 8.36 (m, 2 H), 8.18 (s, 1 H), 8.06 (dd, *J*=8.4, 2.1 Hz, 1 H), 7.82 - 7.93 (m, 2 H), 7.72 - 7.81 (m, 2 H), 7.50 (br t, *J*=9.2 Hz, 1 H), 7.16 - 7.22 (m, 1 H), 4.03 - 4.17 (m, 1 H), 3.87 - 4.02 (m, 1 H), 3.70 (s, 3 H), 2.87 (dq, *J*=14.5, 7.1 Hz, 1 H), 2.48 (br s, 3 H), 1.08 (d, *J*=7.0 Hz, 3 H). MS-ESI *m*/*z:* 544.1 [M+H]⁺.

### Embodiment 2:

### Preparation of the crystal form A, B, C and D of the compound represented by formula (I)

Approximately 50 mg of the compound represented by formula (I) was weighed in a sample bottle and a certain volume of the solvent from the table below was added to prepare suspensions or solutions of different single and mixed solvents.

After the suspension was continuously shaken for 3 days at 40°C, the residual solid was centrifuged and put into a vacuum drying oven and dried under vacuum overnight at 40°C to remove the residual solvent to obtain each crystal form of the compound represented by formula (I).

The solution was placed to evaporate at room temperature and then dried under vacuum at 30°C for 1 day to remove the residual solvent to obtain each crystal form of the compound represented by formula (I).

The results are shown in Table 6.

**Table 6: Screening experiment of different solvent crystal forms of the compound represented by formula (I)**

| **No.** | **Solvent** | **Solvent volume (mL)** | **State** | **Crystal form** |
|---|---|---|---|---|
| 1 | Ethanol | 1.00 | Suspension | Crystal form A |
| 2 | acetone | 0.60 | Solution (solid precipitated after dissolving) | Crystal form C |
| 3 | Ethyl acetate | 1.00 | Suspension | Crystal form A |
| 4 | Methanol: water (2:1) | 1.00 | Solution (solid precipitated after dissolving) | Crystal form B |
| 5 | Ethanol: water (2:1) | 1.00 | Solution (solid precipitated after dissolving) | Crystal form B |
| 6 | Acetone: water (2:1) | 0.86 | Solution (solid precipitated after dissolving) | Crystal form B |
| 7 | Water | 1.50 | Suspension | Crystal form D |
| 8 | Ethanol: water (1:3) | 1.00 | Suspension | Crystal form B |

### Preparation of the crystal form A of the compound represented by formula (I)

1 kg of the crude product of the compound represented by formula (I) was transferred into the reaction kettle with ethyl acetate (7000 mL), heated to 60 °C and stirred until complete dissolution, then the solution was cooled to 40 °C and stirred until a large number of solids were precipitated, followed by continued stirring at a temperature of 40°C overnight and then filtered, which was the crystal form A of the compound represented by formula (I).

### Embodiment 3: Stability test of the crystal form A of the compound represented by formula (I)

The stability of the crystal form A of the compound represented by formula (I) was investigated according to the Guideline for Stability Testing of APIs and Formulations (Chinese Pharmacopoeia 2015 Edition IV General Rule 9001).

5mg of the crystal form A of the compound represented by formula (I) was weighed separately, placed in a dry and clean glass bottle, in duplicate, spread into a thin layer, as a formal test sample, the two samples were placed under the test conditions of influencing factors (60 °C, 92.5% relative humidity) and accelerated conditions (30 °C/65% relative humidity, 40 °C/75% relative humidity and 60 °C/75% relative humidity), the samples were fully exposed, covered with aluminum foil and pierced with small holes. Sampling and analysis were performed on day 5, day 10, and 1 month. Samples placed under illumination (total illumination 1200000 Lux•hr, near ultraviolet 200 w•hr/m²) were completely exposed at room temperature. The experimental results are shown in the following Table 7.

**Table 7: Solid stability test results of the crystal form A of the compound represented by formula (I)**

| Test condition | Time point | Appearance | Crystal form (XRPD) | Content of the crystal form A of the compound represented by formula (I) (%) | Total impurity content (%) |
|---|---|---|---|---|---|
| - | Day 0 | Off-white powder | Crystal form A | 103.08 | 0.14 |
| High temperature (60°C, open) | Day 5 | Off-white powder | Crystal form A | 104.39 | 0.14 |
| | Day 10 | Off-white powder | Crystal form A | 102.00 | 0.14 |
| High humidity (25°C/92.5% relative humidity, open) | Day 5 | Off-white powder | Crystal form A | 103.08 | 0.14 |
| | Day 10 | Off-white powder | Crystal form A | 103.95 | 0.14 |
| In the absence of light | Day 10 | Off-white powder | Crystal form A | 102.57 | 0.15 |
| Illumination (total illumination 1.2×106 Lux•hr/near ultraviolet 200 w•hr/m², open). | Day 10 | Off-white powder | Crystal form A | 103.04 | 0.14 |
| 40°C, relative humidity 75%, open | Day 10 | Off-white powder | Crystal form A | 103.79 | 0.15 |
| | 1 month | Off-white powder | Crystal form A | 103.86 | 0.16 |
| 60°C, relative humidity 75%, open | Day 10 | Off-white powder | Crystal form A | 103.63 | 0.14 |
| | 1 month | Off-white powder | Crystal form A | 103.30 | 0.14 |
| 30°C, relative humidity 65%, open | Day 10 | Off-white powder | Crystal form A | 104.28 | 0.14 |
| | 1 month | Off-white powder | Crystal form A | 101.64 | 0.14 |

Conclusion: The crystal form A of the compound represented by formula (I) has good stability.

### Embodiment 4: Study on the hygroscopicity of the crystal form A of the compound represented by formula (I)

Experimental Materials:
SMS DVS Advantage Dynamic Vapor Sorption instrument

Experimental method:
20 mg of the crystal form A of the compound represented by formula (I) was placed in the DVS sample tray for testing.

Experimental results:
The DVS isotherm plot of the crystal form A of the compound represented by formula (I) is shown in Figure 4, ΔW=0.885%.

Experimental conclusion:
The hygroscopical weight gain of the crystal form A of the compound represented by formula (I) at 25 °C and 80% RH is 0.885%, which is slightly hygroscopic.

### Embodiment 5: Study on the hygroscopicity of the crystal form B of the compound represented by formula (I)

Experimental Materials:
SMS DVS Advantage Dynamic Vapor Sorption System

Experimental method:
20 mg of the crystal form A of the compound represented by formula (I) was placed in the DVS sample tray for testing.

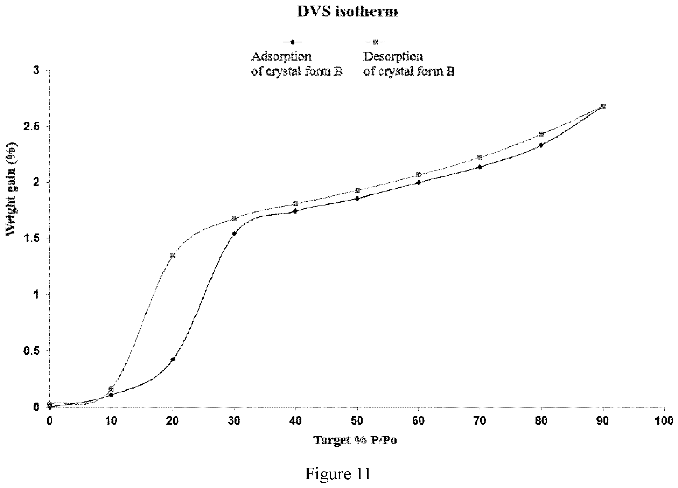

Experimental results:
The DVS isotherm plot of the crystal form B of the compound represented by formula (I) is shown in Figure 11, ΔW=2.332%.

Experimental conclusion:
The hygroscopical weight gain of the crystal form B of the compound represented by formula (I) at 25 °C and 80% RH is 2.332%, which is hygroscopic.

### Experimental embodiment 1. Study on the activity and selectivity of the crystal form A of the compound represented by formula (I) on PI3Kα/β/γ/δ kinase and cell in vitro

The crystal form A of the compound represented by formula (I) has a strong inhibitory effect on both wild-type and mutant PI3Kα kinases. The IC₅₀ of the crystal form A of the compound represented by formula (I) on the inhibition of wild-type PI3Kα, mutant PI3Kα(E545K) and PI3Kα(H1047R) were 1.80, 1.13 and 0.69 nM, respectively. The crystal form A of the compound represented by formula (I) has excellent selectivity to the other three subtypes of PI3K, and its inhibitory activity against PI3Kα is 149/7.44/6.61 times higher than that of PI3Kβ/δ/γ, respectively. The specific experimental method was the same as experimental embodiment 8. Under the same test conditions, the crystal form A of the compound represented by formula (I) showed excellent selectivity for the Akt phosphorylation inhibitory activity of the specific cell line MDA-MB-468/Jeko-1/RAW264.7 with high expression of PI3Kβ/δ/γ, and its inhibitory activity against PI3Kα was 195/23.0/>694 times more than PI3Kβ/δ/γ, respectively, see Table 8 and Table 9 for details. The specific experimental method was the same as experimental embodiment 9.

**Table 8 In vitro activity of the crystal form A of the compound represented by formula (I) on wild-type and mutant PI3Kα kinase, IC₅₀ (nM)**

| Compound | Wild type PI3Kα | Mutant PI3Kα (E545K) | Mutant PI3Kα (H1047R) |
|---|---|---|---|
| Crystal form A of the compound represented by formula (I): | 1.80 | 1.13 | 0.69 |

**Table 9 Selectivity of the crystal form A of the compound represented by formula (I) on PI3Kα/β/γ/δ kinase.**

| Compound | PI3Kα/β | PI3Kα/δ | PI3Kα/γ |
|---|---|---|---|
| Crystal form A of the compound represented by formula (I): | 149 | 7.44 | 6.61 |

### Experimental embodiment 2. In vivo pharmacodynamic study of the crystal form A of the compound represented by formula (I) in an HR+/HER2+ human breast cancer BT-474 (PIK3CA amplified) nude mouse subcutaneous xenograft tumor model

The phenotype of human BT-474 breast cancer cells is HR+/HER2+, and it has PIK3CA amplification. This experiment evaluated the efficacy of the crystal form A of the compound represented by formula (I) in a human breast cancer xenograft tumor model, using BYL-719 as a reference.

Cell culture: Human breast cancer BT474 cells were cultured *in vitro* in a single layer, the culture condition was Hybri-Care medium with 10% fetal bovine serum and the cells were incubated in 5% CO₂ at 37°C. Digestion and passage treatment with trypsin-EDTA was carried out twice a week. When the cell saturation was 80%-90% and the number reached the requirement, the cells were collected, counted and inoculated.

Animals: BALB/c nude mice, female, 6-8 weeks old, weighing 18-20g. A total of 85 animals were required, and provided by Shanghai Bikai experimental animal Co., Ltd.

Tumor inoculation: Estrogen tablets (0.36mg/ tablet) were subcutaneously inoculated on the left back of each mouse, three days later, 0.2 mL (10×10⁶ cells) of BT474 cells (with matrigel in a volume ratio of 1:1) were subcutaneously inoculated on the right back of each mouse, group administration was started when the average tumor volume reached 150 mm³-200 mm³.

After oral administration once a day for 20 days, there were statistically significant differences between BYL-719 (40 mg/kg), the crystal form A of the compound represented by formula (I) (40 mg/kg) and vehicle control group (P values were 0.003 and 0.001, respectively), and their T/C (relative tumor proliferation rate T/C (%) = TRTV / CRTV × 100 % (TRTV: mean relative tumor volume in the treatment group; CRTV: mean relative tumor volume in the negative control group)) were 29.39% and 21.16%, respectively, and TGI (tumor growth inhibition rate, TGI (%) = [1-(mean tumor volume at the end of the administration in a treatment group - mean tumor volume at the beginning of the administration in that treatment group)]/(mean tumor volume at the end of treatment in the vehicle control group - mean tumor volume at the beginning of treatment in the vehicle control group)]×100%) were 95.31% and 105.65%, respectively. Compared with the vehicle control group, there were statistically significant differences (p values were 0.034 and 0.007 respectively) between the crystal form A of the compound represented by formula (I) (10 mg/kg) and the crystal form A of the compound represented by formula (I) (20 mg/kg), with T/C sum of 50.18%, 37.92% and TGI of 65.34%, 80.21%, respectively. Tumor-bearing mice in each administration group had good tolerance to the tested compounds. BYL-719 (40 mg/kg) group and the crystal form A of the compound represented by formula (I) (40 mg/kg) group had significant anti-tumor effects, the crystal form A of the compound represented by formula (I) (10 mg/kg) group, the crystal form A of the compound represented by formula (I) (20 mg/kg) group had a strong anti-tumor effect. The anti-tumor effect of the crystal form A of the compound represented by formula (I) showed a certain dose dependence in the dose set in this experiment, and the dose to play the effect was 10 mg/kg. The specific results are shown in Table 10.

On the last day of administration, the plasma and tumor tissues of animals were collected for PK test, the PK results showed that with the increase of administration dose, the plasma exposure of the crystal form A of the compound represented by formula (I) increased linearly. 0.5-1 hours after administration, the blood concentration reached the peak. The plasma exposure at the dose to play the effect was 69 300 nM^{∗}h.

**Table 10 Results of in vivo efficacy study of the crystal form A of the compound represented by formula (I) in human breast cancer BT-474 nude mouse subcutaneous xenograft model**

| Compound | TGI% | T/C% |
|---|---|---|
| BYL-719 (40 mpk) | 95.31% | 29.39% |
| Crystal form A of the compound represented by formula (I) (40 mpk) | 105.65% | 21.16% |
| Crystal form A of the compound represented by formula (I) (20 mpk) | 80.21% | 37.92% |
| Crystal form A of the compound represented by formula (I) (10 mpk) | 65.34% | 50.18% |

### Experimental embodiment 3. In vivo pharmacodynamic study of the crystal form A of the compound represented by formula (I) in HR+/HER2+ human breast cancer T47D (PIK3CA H1047R mutation) nude mouse subcutaneous xenograft tumor model

The phenotype of human T47D breast cancer cells is HR+/HER2-, and it carries PIK3CAH1047R mutation. This experiment evaluated the efficacy of the crystal form A of the compound represented by formula (I) in a human breast cancer xenograft tumor model.

Cell culture: Human breast cancer T47D cells were cultured *in vitro* in a single layer, the culture condition was RPMI-1640 medium with 0.2 U/mL bovine insulin and 10% fetal bovine serum, and the cells were incubated in 5% CO₂ at 37°C. Digestion and passage treatment with trypsin-EDTA was carried out twice a week. When the cell saturation was 80%-90% and the number reached the requirement, the cells were collected, counted and inoculated.

Animals: BALB/c nude mice, female, 6-8 weeks old, weighing 18-20g. A total of 75 animals were required, and provided by Shanghai Bikai experimental animal Co., Ltd or other qualified suppliers.

Tumor inoculation: Estrogen tablets (0.18mg/ tablet) were subcutaneously inoculated on the left back of each mouse. Three days later, 0.2 mL (10×10⁶ cells) of T47D cells (with matrigel in a volume ratio of 1:1) were subcutaneously inoculated on the right back of each mouse, group administration was started when the average tumor volume reached 150mm³ -200 mm³.

After oral administration once a day for 42 days, there were statistically significant differences (p value was <0.001) between the crystal form A of the compound represented by formula (I) (40 mg/kg) and the vehicle control group, with T/C of 37.91%, and TGI of 84.71%. Compared with the vehicle control group, there were statistically significant differences (p values were 0.005 and 0.002 respectively) between the crystal form A of the compound represented by formula (I) (10 mg/kg) and the crystal form A of the compound represented by formula (I) (20 mg/kg), with T/C of 50.40%, 44.70% and TGI of 67.58%, 72.56%, respectively. Tumor-bearing mice in each administration group had good tolerance to the tested compounds. The crystal form A of the compound represented by formula (I) (40 mg/kg) group had significant anti-tumor effects, the crystal form A of the compound represented by formula (I) (10 mg/kg) group, the crystal form A of the compound represented by formula (I) (20 mg/kg) group had a strong anti-tumor effect. The anti-tumor effect of the crystal form A of the compound represented by formula (I) showed a certain dose dependence in the dose set in this experiment, and the dose to play the effect was 10 mg/kg. The specific results are shown in Table 11.

**Table 11 Results of in vivo efficacy study of the crystal form A of the compound represented by formula (I) in human breast cancer T47D nude mouse subcutaneous xenograft model**

| Compound | TGI% | T/C% |
|---|---|---|
| Crystal form A of the compound represented by formula (I) (40 mpk) | 84.71% | 37.91% |
| Crystal form A of the compound represented by formula (I) (20 mpk) | 72.56% | 44.70% |
| Crystal form A of the compound represented by formula (I) (10 mpk) | 67.58% | 50.40% |

### Experimental embodiment 4. In vivo pharmacodynamic study of the crystal form A of the compound represented by formula (I) in human ovarian cancer SKOV-3 (PIK3CA H1047R mutation) nude mouse subcutaneous xenograft tumor model

Human SKOV-3 ovarian cancer cells with PIK3CA H1047R mutation. This experiment evaluated the efficacy of the crystal form A of the represented by formula (I) in a human ovarian cancer xenograft tumor model.

Cell culture: Human ovarian cancer SKOV-3 cells were cultured *in vitro* in a single layer, the culture condition was RPMI-1640 medium with 10% fetal bovine serum, and the cells were incubated in 5% CO₂ at 37°C. Digestion and passage treatment with trypsin-EDTA was carried out twice a week. When the cell saturation was 80%-90% and the number reached the requirement, the cells were collected, counted and inoculated.

Animals: BALB/c nude mice, female, 6-8 weeks old, weighing 18-20g. A total of 67 animals were required, and provided by Beijing Vital River Biotechnology Co., Ltd.

Tumor inoculation: 0.2 mL (10×10⁶ cells) of SKOV-3 cells (with matrigel in a volume ratio of 1:1) were subcutaneously inoculated on the right back of each mouse, group administration was started when the average tumor volume reached 150mm³ -200 mm³.

After oral administration once a day for 28 days, there were statistically significant differences (p value was <0.001) between the crystal form A of the compound represented by formula (I) (40 mg/kg) and the vehicle control group, with T/C of 37.79%, and TGI of 69.16%. Compared with the vehicle control group, there were statistically significant differences (p values were 0.041 and 0.005 respectively) between the crystal form A of the compound represented by formula (I) (10 mg/kg) group and the crystal form A of the compound represented by formula (I) (20 mg/kg) group, with T/C sum of 69.17%, 60.61% and TGI of 30.45%, 41.42%, respectively. Tumor-bearing mice in each administration group had good tolerance to the tested compounds. The crystal form A of the compound represented by formula (I) (40 mg/kg) group had significant anti-tumor effects, the crystal form A of the compound represented by formula (I) (10 mg/kg), the crystal form A of the compound represented by formula (I) (20 mg/kg) had a certain anti-tumor effect. The anti-tumor effect of the crystal form A of the compound represented by formula (I) showed a certain dose dependence in the dose set in this experiment. The specific results are shown in Table 12.

**Table 12 Results of in vivo efficacy study of the crystal form A of the compound represented by formula (I) in human ovarian cancer SKOV-3 nude mouse subcutaneous xenograft model**

| Compound | TGI% | T/C% |
|---|---|---|
| Crystal form A of the compound represented by formula (I) (40 mpk) | 69.16% | 37.79% |
| Crystal form A of the compound represented by formula (I) (20 mpk) | 41.42% | 60.61% |
| Crystal form A of the compound represented by formula (I) (10 mpk) | 30.45% | 69.17% |

### Experimental embodiment 5. Sprague Dawley (SD) rat absorption test of the crystal form A of the compound represented by formula (I)

SD rats were given the crystal form A of the compound represented by formula (I) by single or multiple oral gavage and single intravenous injection respectively, with 6 rats in each group, half male and half female. According to the results of pharmacodynamic and toxicological experiments, the single oral gavage doses were 3, 10 and 30 mg/kg respectively; the multiple doses were 10 mg/kg, once a day for 7 consecutive days; the single intravenous dose was 1 mg/kg. According to the drug plasma concentration-time curve, the pharmacokinetic parameters were calculated. The results of male rats are shown in Table 13, and the results of female rats are shown in Table 14. In this experiment, SD rats were provided by Beijing Vital River Experimental Animal Technology Co., Ltd., and were divided into 4 groups (3/ sex/group) according to their similar weight. The vehicle for the intravenous group was 5% HP-betaCD/5% Solutol aqueous solution (pH=8); the vehicle for the oral group was 0.5% MC/0.2% Tw80 aqueous solution. Plasma samples were collected by jugular vein puncture in rats.

After a single intravenous administration of 1 mg/kg, the plasma clearance rates (CL) of the crystal form A of the compound represented by formula (I) in male and female SD rats were 1.79 ± 0.457 and 3.12 ± 0.431 mL/min/kg respectively, and the steady-state apparent distribution volumes (Vdss) were 0.265 ± 0.0500 and 0.257 ± 0.0227 L/kg respectively, the elimination half-lives (t_{1/2}) were 3.26 ± 1.13 h and 1.63 ± 0.809 h, and the system exposures (AUC₀₋ₗₐₛₜ) were 17400±4790 nM^{∗}h and 9890±1410 nM^{∗}h.

After a single oral administration of 3 mg/kg of the crystal form A of the compound represented by formula (I) to male SD rats, the bioavailability was 34.7%. After a single oral administration of 3, 10 or 30 mg/kg of the crystal form A of the compound represented by formula (I) to male SD rats, the AUC₀₋ₗₐₛₜ were 10300 ± 4600, 23700 ± 721 and 45300 ± 10900 nM^{∗}h, respectively, reaching peak concentrations (Cₘₐₓ) were 4770 ± 1010, 6800 ± 583, and 14500 ± 4730 nM, respectively, reaching peak times appeared at 0.417 ± 0.144 h, 0.500 ± 0.000 h, and 0.667 ± 0.289 h after administration. After a single oral administration of 3 mg/kg of the crystal form A of the compound represented by formula (I) to female SD rats, the bioavailability was 53.1%. After a single oral administration of 3, 10 or 30 mg/kg of the crystal form A of the compound represented by formula (I) to female SD rats, the AUC₀₋ₗₐₛₜ were 27700 ± 8720, 60900 ± 10900 and 177000 ± 48000 nM^{∗}h, respectively, reaching peak concentrations (Cₘₐₓ) were 6390 ± 1710, 12100 ± 3690, and 39100 ± 7 310 nM, respectively, reaching peak times were 0.500 ± 0.000 h, 0.667 ± 0.289 h, and 0.500 ± 0.000 h.

After intragastric administration of SD rats with 10 mg/kg each time for 7 consecutive days, the Cₘₐₓ of male rats on the day 1 and day 7 were 6800 ± 583 and 13900 ± 1610 nM, respectively, and the AUC₀₋ₗₐₛₜ were 23700 ± 721 and 48500 ± 4640 nM^{∗}h. The Cₘₐₓ of female rats on the day 1 and day 7 were 12100 ± 3690 and 20500 ± 4600 nM, respectively, and the AUC₀₋ₗₐₛₜ were 60900 ± 10900 and 86000 ± 19900 nM^{∗}h.

**Table 13 Average pharmacokinetic parameters of the crystal form A of the compound represented by formula (I) after single or multiple administration to male SD rats (n=3)**

| **Group** | **1** | | **2** | | **3 (Day 1)** | | **3 (Day 7)** | | **4** | |
|---|---|---|---|---|---|---|---|---|---|---|
| **Route of administration** | **vein** | | **oral** | | **oral** | | **oral** | | **oral** | |
| **Dose (mg/kg)** | **1** | | **3** | | **10** | | **10** | | **30** | |
| **Pharmacokinetic parameters** | **Avera ge value** | **stand ard devia tion** | **Aver age valu e** | **stand ard devia tion** | **Aver age valu e** | **stand ard devia tion** | **Aver age valu e** | **stand ard devia tion** | **Aver age valu e** | **stand ard devia tion** |
| **C₀ or Cₘₐₓ (nM)** | | | 4770 | 1010 | 6800 | 583 | 1390 | 1610 | 1450 | 4730 |
| | 12700 | 1590 | | | | | 0 | | 0 | |
| **Tₘₐₓ (h)** | -- | -- | 0.417 | 0.144 | 0.500 | 0.000 | 0.667 | 0.289 | 0.667 | 0.289 |
| **t_{1/2} (h)** | 1.63 | 0.809 | 2.26 | 0.593 | 2.49 | 0.492 | 3.95 | 1.49 | 2.61 | 0.509 |
| **Vdss (L/kg)** | | 0.022 | -- | -- | -- | -- | -- | -- | -- | -- |
| | 0.257 | 7 | | | | | | | | |
| **CL (mL/min/kg)** | 3.12 | 0.431 | -- | -- | -- | -- | -- | -- | -- | -- |
| **AUC₀₋ₗₐₛₜ (nM^{∗}h)** | | | 1030 | 4600 | 2370 | 721 | 4850 | 4640 | 4530 | 10900 |
| | 9890 | 1410 | 0 | | 0 | | 0 | | 0 | |
| **Oral bioavailability (%)** | -- | -- | 34.7 | / | / | / | / | / | / | / |

Tₘₐₓ: time to reach the maximum concentration of the drug *in vivo* after oral administration; Co: initial concentration of the drug *in vivo* after intravenous administration; "": there is no such parameter for the corresponding administration method"; "/": not calculated.

**Table 14 Average pharmacokinetic parameters of the crystal form A of the compound represented by formula (I) after single or multiple administration to female SD rats (n=3)**

| **Group** | **1** | | **2** | | **3 (Day 1)** | | **3 (Day 7)** | | **4** | |
|---|---|---|---|---|---|---|---|---|---|---|
| **Route of administration** | **vein** | | **oral** | | **oral** | | **oral** | | **oral** | |
| **Dose (mg/kg)** | **1** | | **3** | | **10** | | **10** | | **30** | |
| **Pharmacokinetic parameters** | **Aver age valu e** | **stand ard devia tion** | **Aver age valu e** | **stand ard devia tion** | **Aver age valu e** | **stand ard devia tion** | **Aver age valu e** | **stand ard devia tion** | **Aver age valu e** | **stand ard devia tion** |
| **C₀ or Cₘₐₓ (nM)** | 1420 | | 6390 | 1710 | 1210 | 3690 | 2050 | 4600 | 3910 | 7310 |
| | 0 | 586 | | | 0 | | 0 | | 0 | |
| **Tₘₐₓ (h)** | - | - | 0.500 | 0.000 | 0.667 | 0.289 | 0.500 | 0.000 | 0.500 | 0.000 |
| **t_{1/2} (h)** | 3.26 | 1.13 | 2.71 | 0.129 | 2.92 | 0.378 | 3.74 | 0.923 | 2.72 | 0.220 |
| **Vdss (L/kg)** | | 0.050 | - | - | - | - | - | - | - | - |
| | 0.265 | 0 | | | | | | | | |
| **CL (mL/min/kg)** | 1.79 | 0.457 | - | - | - | - | - | - | - | - |
| **AUC₀₋ₗₐₛₜ (nM^{∗}h)** | 1740 | | 2770 | 8720 | 6090 | 10900 | 8600 | 19900 | 1770 | 48000 |
| | 0 | 4790 | 0 | | 0 | | 0 | | 00 | |
| **Oral bioavailability (%)** | - | - | 53.1 | / | / | / | / | / | / | / |

Tₘₐₓ: time to reach the maximum concentration of the drug *in vivo* after oral administration; Co: initial concentration of the drug *in vivo* after intravenous administration; "": there is no such parameter for the corresponding administration method"; "/": not calculated.

### Experimental embodiment 6: Beagle dog absorption test of the crystal form A of the compound represented by formula (I)

Beagle dogs were given the crystal form A of the compound represented by formula (I) by single or multiple oral administration and single intravenous injection respectively, with 6 dogs in each group, half male and half female. The single oral doses were 0.3, 1 and 3 mg/kg respectively; the multiple doses were 1 mg/kg, once a day for 7 consecutive days; the single intravenous dose was 0.3 mg/kg. According to the drug plasma concentration-time curve, the pharmacokinetic parameters were calculated, the results are shown in Table 15. The vehicle for drug preparation in group 1 was 5% HP-beta-CD, 5% solutol, pH = 8 aqueous solution, the vehicles for groups 2, 3, 4 were 0.5% MC, 0.2% Tween80 aqueous solution.

**Table 15 Average pharmacokinetic parameters of the crystal form A of the compound represented by formula (I) after single or multiple administration to male and female Beagle dogs (n=6)**

| **Group** | **1** | | **2** | | **3 (day 1)** | | **3 (day 7)** | | **4** | |
|---|---|---|---|---|---|---|---|---|---|---|
| **Route of administration** | **vein** | | **oral** | | **oral** | | **oral** | | **oral** | |
| **Dose (mg/kg or mg/dog)** | **0.3** | | **0.3** | | **1** | | **1** | | **3** | |
| **Pharmacokinetic parameters** | **Aver age valu e** | **stand ard devia tion** | **Aver age valu e** | **stand ard devia tion** | **Aver age valu e** | **stand ard devia tion** | **Aver age valu e** | **stand ard devia tion** | **Aver age valu e** | **stand ard devia tion** |
| **C₀ or Cₘₐₓ (nM)** | 512 | 215 | 158 | 68.4 | 656 | 30.7 | 850 | 106 | 1880 | 274 |
| **Tₘₐₓ (h)** | - | - | 2.00 | 1.10 | 1.67 | 0.516 | 1.33 | 0.516 | 3.08 | 1.50 |
| **T_{1/2} (h)** | 6.32 | 1.62 | 6.92 | 3.16 | 5.00 | 1.44 | 5.18 | 0.487 | 6.55 | 1.76 |
| **Vdss (L/kg)** | 2.47 | 0.391 | - | - | - | - | - | - | - | - |
| **CL (mL/min/kg)** | 6.18 | 1.49 | - | - | - | - | - | - | - | - |
| **AUC₀₋ₗₐₛₜ (nM^{∗}h)** | 1470 | 353 | 1290 | 715 | 4980 | 946 | 5880 | 697 | 1480 | 2510 |
| | | | | | | | | | 0 | |
| **Oral bioavailability (%)** | - | - | 87.8 | / | / | / | / | / | / | / |

Tₘₐₓ: time to reach the maximum concentration of the drug *in vivo* after oral administration; Co: initial concentration of the drug *in vivo* after intravenous administration; "": there is no such parameter for the corresponding administration method"; "/": not calculated.

After a single intravenous injection of 0.3 mg/kg of the crystal form A of the compound represented by formula (I) to male and female Beagle dogs, the plasma clearance (CL) of the crystal form A of the compound represented by formula (I) was 6.18±1.49 mL/min/kg, the steady-state apparent volume of distribution (Vdss) was 2.47±0.391 L/kg, and the elimination half-life (t_{1/2}) and the area under the plasma concentration curve (AUC₀₋ₗₐₛₜ) from 0 to the last quantifiable time point were respectively 6.32±1.62 h and 1470±353 nM^{∗}h, respectively.

After a single oral administration of 0.3 mg/kg of the crystal form A of the compound represented by formula (I) to male and female beagle dogs, the bioavailability was 87.8%. After a single oral administration of 0.3, 1 and 3 mg/kg of the crystal form A of the compound represented by formula (I) to male and female beagle dogs, AUC₀₋ₗₐₛₜ were 1290 ± 715, 4980 ± 946 and 14800 ± 2510 nM^{∗}h, respectively, reaching peak concentrations (Cₘₐₓ) were 158 ± 68.4, 656 ± 30.7, and 1880 ± 274 nM, respectively, reaching peak times appeared at 2.00 ± 1.10 h, 1.67 ± 0.516 h, and 3.08 ± 1.50 h after administration. T_{1/2} were 6.92 ± 3.16, 5.00 ± 1.44 and 6.55 ± 1.76 h, respectively.

Male and female beagle dogs were given 1 mg/kg of the crystal form A of the compound represented by formula (I) orally for 7 consecutive days, 1 day after the administration, AUC₀₋ₗₐₛₜ was 4980±946nm ^{∗} h, Cₘₐₓ was 656±30.7nm and T_{1/2} was 5.00±1.44h. 7 days after the administration for, AUC₀₋ₗₐₛₜ was 5880±697nm ^{∗} h, Cₘₐₓ was 850±106nm and T_{1/2} was 5.18±0.487 h.

According to the above-mentioned administration mode and calculation mode, after a single oral administration of 0.3 mg/kg of the amorphous compound represented by formula (I) to male and female beagle dogs, the bioavailability was 71.2%.

Conclusion: The crystal form A of the compound represented by formula (I) has good oral bioavailability, low clearance, high systemic exposure and excellent pharmacokinetic properties in both animal species.

### Experimental embodiment 7. Western blot analysis of p-AKT protein expression level in BT474 tumor tissue samples

Experimental method:
7.1 Protein extraction and quantification
   1) Quick-frozen tissue samples were taken out from the refrigerator at-80°C.
   2) Operating on dry ice, cutting part of the tissues (about 30mg), putting them into a 2mL centrifuge tube with steel balls, and adding 500µL of cell lysate RIPA (1% protease inhibitor and phosphatase inhibitor had been newly added).
   3) Tissue was broken for 5 minutes using the highest frequency of Tissuelyser LT.
   4) The tissue lysate was placed on ice for 30 minutes.
   5) Centrifuging at 12,000 rpm at 4°C for 10 minutes and removing the supernatant into a new 1.5 mL centrifuge tube.
   6) The protein was quantified by BCA quantitative kit.
   7) According to the quantitative results, the protein sample for loading was prepared, the protein concentration of the sample was unified to 2µg/µL, LDS loading buffer (4X) and sample reducing agent (10X) were added, and the sample was heated at a constant temperature of 100°C for 10 minutes.
   8) Western blotting, or storing the denatured samples in a refrigerator at -80°C.
7.2 Western blot
   1) The sample for loading was thawed.
   2) Loading: in SDS-PAGE gel, 10µL of sample was loaded in each well (the loading amount depends on their own needs).
   3) Electrophoresis: 80 volts, 30 minutes, followed by 120 volts, 90 minutes.
   4) Membrane transfer: iBlot2 membrane transfer kit and membrane transfer instrument were used, and P3 program was run for 7 minutes.
   5) After the membrane transfer was finished, the membrane was cut according to the molecular weight of the protein to be detected, the membrane was washed with 1xTBST for 3 times and 5 minutes each time, and was shaken at room temperature.
   6) Sealing: The membrane was placed in a sealing solution (5% skimmed milk prepared with 1xTBST), and was shaken at room temperature for 1 hour.
   7) Membrane was washed with 1xTBST for 3 times and 5 minutes each time, and was shaken at room temperature.
   8) Incubation of primary antibody: primary antibody with proper dilution was added (diluted with 5% skim milk or bovine serum albumin prepared by 1xTBST), kept at 4°C overnight, and was shaken slowly.
   9) Membrane was washed with 1xTBST for 3 times and 10 minutes each time, and was shaken at room temperature.
   10) Incubation of secondary antibody: secondary antibody with proper dilution was added, and was shaken slowly at room temperature for 1 hour.
   11) Membrane was washed with 1xTBST for 3 times and 10 minutes each time, and was shaken at room temperature.
   12) Chemiluminescence: HRP substrate in West Femto hypersensitive chemiluminescence kit was added to the membrane.
   13) Chemiluminescence was detected and photographed on the Tanon 5200 multi machine.

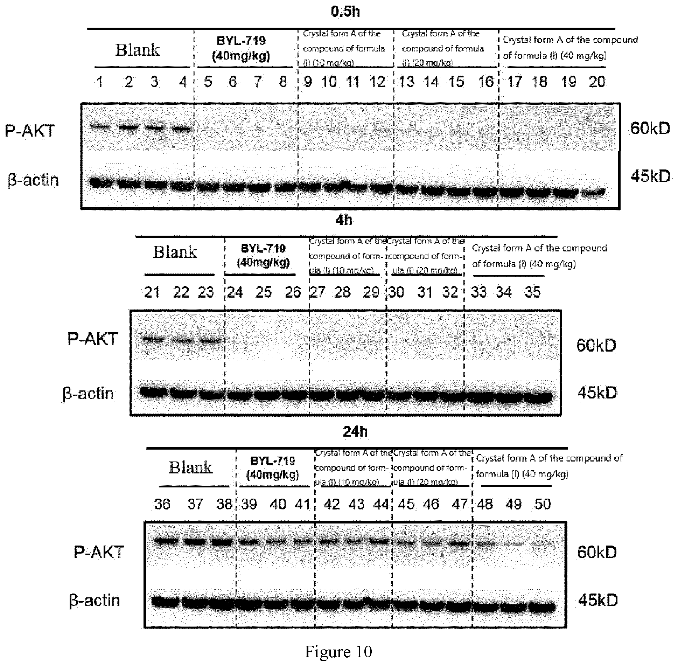

The results are shown in Figure 10. PD (*in vivo* pharmacodynamic biomarker detection) results show that the crystal form A of the compound represented by formula (I) can significantly inhibit Akt phosphorylation level downstream of PI3K in BT-474 nude mouse transplanted tumor model, and show a certain time and dose dependence.

### Experimental embodiment 8: In vitro enzymatic activity test of the compound represented by formula (I)

The lipid kinase reaction was carried out in a suitable substrate and under ATP conditions, then the activity of the kinase was detected by ADP-Glo^{™} kit in two steps. Step 1: the kinase reaction was terminated, in which the residual ATP was completely removed and only ADP was retained; step 2: kinase detection reagent was added to convert ADP to ATP, accompanied by fluorescein/luciferase reaction. Finally, the fluorescence value output was converted into kinase activity. Conditions for testing PI3K enzyme activity are shown in Table 16.

**Table 16 Conditions for testing PI3K enzyme activity**

| Subtype | Final concentration of enzyme | ATP (µM) | PIP2:3PS (µM) | Reaction time (min) |
|---|---|---|---|---|
| PI3Kα | 0.2 nM | 40 | 50 | 120 |
| PI3Kβ | 0.6 nM | 40 | 50 | 120 |
| PI3Kδ | 0.25 nM | 40 | 50 | 120 |
| PI3Kγ | 0.4 nM | 25 | 50 | 120 |

Experimental materials and equipment:
1) Enzyme: PI3Kα Millipore #14-602-K
   PI3Kβ Promega #V1751
   PI3Kδ Millipore #14-604-K
   PI3K γ Millipore #14-558-K
2) Kit: ADP-Glo^{™} lipid kinase and PIP2:3PS kit (Promega #V1792)
   The reagent kit contains: 1 mM PIP2:3PS, 10× lipid dilution buffer, 1 M magnesium chloride, 10 mM ATP, 10 mM ADP, ADP-Glo reagent, detection buffer and detection substrate.
3) Reaction plate: OptiPlate-384, white and transparent (PerkinElmer #6007299)

Reagent preparation:
1) 10× reaction buffer: 500 mM HEPES, pH 7.5, 500 mM NaCl, 9 mM MgCl₂; BSA: 10% stock solution, self-made
2) Final test system conditions: 1× reaction system: 50 mM of HEPES, 50 mM of NaCl, 3mM of MgCl₂, 0.01%BSA (freshly prepared on the day of experiment), 1% DMSO (v/v) +/compound
3) Reaction system: 3 µL of mixture of enzyme and substrate (1:1)+ 2 µL of ATP/MgCl₂ mixture+5 µL of ADP-Glo reagent+10 µL of detection reagent.

The specific experimental operations were as follows:
1) compound dilution: 50 nL 100× compound /DMSO was transferred to the test well plate with Echo
   - For PI3Kα, compounds were three-fold diluted from the highest concentration of 0.111 mM for a total of 10 concentrations.
   - For PI3Kβ/PI3Kδ/PI3Kγ, compounds were tripled diluted from the highest concentration of 1.11 mM for a total of 10 concentrations.
2) Kinase reaction:
   (1) The compound to be tested was prepared, and 50 nL of 100-plus compound solution or DMSO was added to the corresponding well plate.
   (2) 3.33× reaction buffer was prepared
   (3) 3.33×PIP2:3PS was prepared, and PIP2:3PS was vortex thawed at least 1 minute before use
   (4) 2.5 mM containing 5.25 mM MgCl₂ was prepared
   (5) 3.33× PI3Kα/PI3Kβ/PI3Kδ/PI3Kγ solution was prepared
   (6) Lipid kinase solution and PIP2:3PS solution were mixed in a volume ratio of 1:1
   (7) 3.33 ×Lipid kinase buffer and PIP2:3PS solution were mixed in a volume ratio of 1:1
   (8) 3 µL of a mixed solution of buffer and PIP2:3PS was added to columns 1 and 2 of the well plate
   (9) 3 µL of a mixed solution of the enzyme and PIP2:3PS was added to the wells of the well plate except for columns 1 and 2, and centrifuged for 10 s (1000 rpm). The solution was incubated at 23°C for 20 min.
   (10) 2 µL of 2.5n1000 rpm₂ was added and shaken well
   (11) The well plate was covered and shaken well for about 30 s, then the well plate was incubated at 23°C for 2 h
   (12) 5 µL of ADP-Glo reagent containing 10 mM MgCl₂ was added
   (13) Centrifuging at 1000 rpm for 10 s, the well plate was covered, shaken for about 30 s, and incubated at 23°C for 60 min
   (14) 10 µL kinase detection reagent was added
   (15) The well plate was centrifuged at 1000 rpm for 10 s, and then incubated at 23°C for 60 min
   (16) Fluorescence values were measured on the Envision instrument.

### Experimental embodiment 9: In vitro cell activity test of the compound represented by formula (I)

ELISA method was used to measure the inhibition level of the compounds to be tested on the phosphorylation of Akt, which is the downstream protein of PI3K in signal pathway in MCF7 cell line to reflect the cell activity of the compounds.

Cell culture medium: complete cell culture medium (RPMI 1640+10% serum +1% L-glutamine +1% double antibody)

Serum-free medium (without serum, RPMI 1640+1% L-glutamine +1% double antibody)

Specific operation steps were as follows:
(1) MCF7 cells (ATCC^{®} HTB-22^{™}) were inoculated into a 96-well plate with 100 µL (2.5 10⁴ cells per well) of complete cell culture medium, and incubated at 37°C and 5% CO₂ for 24 hours.
(2) The complete cell culture medium was replaced with 100 µL serum-free medium, and incubated overnight in starvation
(3) The compound (the initial concentration of the compound was 1 mM, which was subjected to a three-fold dilution in 10 concentrations. Then, each concentration of the compound was diluted 100-fold with serum-free medium), and 25 µL of the diluted compound was added to the well plate containing cells.
(4) The cells were incubated at 37°C and 5% CO₂ for 2 h
(5) 10 µg/mL insulin (Sigma #I9278-5mL) was used to stimulate the cells in the well plate, incubated for 30 min, and then centrifuged at 1000 rpm for 5 min at room temperature.
(6) 250 µL 1× balanced salt solution (Invitrogen, #14065-056, 4 °C, containing 1mM/L Na₃VO₄) was added to each well to wash the cells once
(7) 100 µL of lysis buffer (tris(hydroxymethyl)aminomethane hydrochloride, Invitrogen, #15567-1000 ml) was added to each well and shaken at 4 °C for 60 min, and then centrifuged at 4000 rpm for 10 min at 4 °C
(8) The subsequent steps were performed according to the ELISA kit (TGR BioSciences #EKT002) instructions.

The results are shown in Table 17.

**Table 17 In vitro screening test results of compound represented by formula (I)**

| **Compound** | PI3Kα IC50 (nM) | PI3Kβ IC50 (nM) | PI3Kδ IC50 (nM) | PI3Kγ IC50 (nM) | MCF7 Cell IC₅₀ (nM) |
|---|---|---|---|---|---|
| **R011 (A2)** | 74.5 | 168 | / | / | 230 |
| **R012 (A10)** | 13.9 | 67.9 | / | / | >1000 |
| Compound represented by formula (I) | 1.75 | 192 | 25.3 | 19.8 | 35.3 |

| | | | | | |
|---|---|---|---|---|---|
| "/": not calculated. | | | | | |

Conclusion: The compound of formula (I) can inhibit the activity of PI3K kinase and has high subtype selectivity for PI3Kβ/γ/δ. In addition, the phosphorylation level of Akt, which is downstream of PI3K, can be well inhibited in cells.

## Claims

1. A crystal form A of a compound represented by formula (I), wherein: 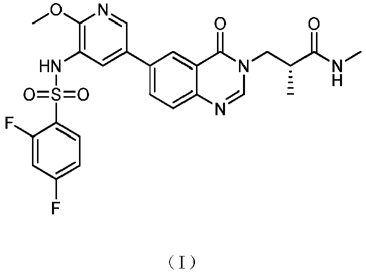
the X-ray powder diffraction pattern thereof has characteristic diffraction peaks at the following angles of 2θ: 4.8 ± 0.2°, 12.6 ± 0.2°, and 17.3±0.2°.
or, the X-ray powder diffraction pattern of the crystal form A has characteristic diffraction peaks at the following angles of 2θ: 4.8±0.2°, 5.7±0.2°, 6.3±0.2°, 11.5±0.2°, 12.6±0.2°, 13.5±0.2°, 17.3±0.2° and 21.5±0.2°;
or, the X-ray powder diffraction pattern of the crystal form A has characteristic diffraction peaks at the following angles of 2θ: 4.8 ± 0.2°, 5.7 ± 0.2°, 6.3 ± 0.2°, 10.1 ± 0.2°, 11.5 ± 0.2°, 12.6 ± 0.2°, 13.5 ± 0.2°, 15.8 ± 0.2°, 17.3 ± 0.2°, 19.2 ± 0.2°, and 21.5 ± 0.2°.
or, in the X-ray powder diffraction pattern thereof, the angles of 2θ are shown in the following table:
| Angles of 2θ (°) | Relative intensity (%) | Angles of 2θ (°) | Relative intensity (%) |
|---|---|---|---|
| 4.8 | 11.5 | 20.3 | 1.4 |
| 5.7 | 3.3 | 20.6 | 0.7 |
| 6.3 | 3.8 | 21.5 | 14.6 |
| 8.0 | 1.3 | 22.8 | 1.6 |
| 9.6 | 1.7 | 23.1 | 0.7 |
| 10.1 | 2.5 | 24.0 | 0.9 |
| 10.6 | 1.5 | 24.3 | 2.5 |
| 11.5 | 3.3 | 25.4 | 5 |
| 12.6 | 100 | 26.7 | 2.7 |
| 13.5 | 4.3 | 27.2 | 0.8 |
| 15.8 | 2 | 29.2 | 2.3 |
| 17.3 | 13.4 | 32.1 | 0.6 |
| 19.2 | 5.3 | 33.1 | 0.9 |
| 19.7 | 0.8 | 33.4 | 1.2 |

2. The crystal form A according to claim 1, wherein the X-ray powder diffraction pattern thereof is shown in Figure 1;
and/or, the differential scanning calorimetry thermogram thereof has an endothermic peak with onset at 195.5±3.0 °C;
and/or, the DSC thermogram thereof is shown in Figure 2;
and/or, the thermogravimetric analysis curve thereof has a weight loss of 0.16% occurred at 151.6±3.0 °C;
and/or, the TGA thermogram thereof is shown in Figure 3.

3. A crystal form B of the compound represented by formula (I), wherein, 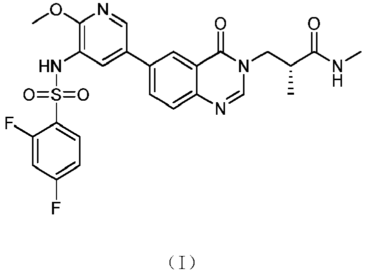
the X-ray powder diffraction pattern thereof has characteristic diffraction peaks at the following angles of 2θ: 5.0 ± 0.2°, 9.9 ± 0.2°, and 12.3±0.2°;
or, the X-ray powder diffraction pattern thereof has characteristic diffraction peaks at the following angles of 2θ: 5.0±0.2°, 9.9±0.2°, 12.3±0.2°, 14.9±0.2°, 20.2±0.2°, 24.4±0.2°, 27.1±0.2° and 30.1±0.2°;
or, in the X-ray powder diffraction pattern thereof, the angles of 2θ are shown in the following table:
| Angles of 2θ (°) | Relative intensity (%) | Angles of 2θ (°) | Relative intensity (%) |
|---|---|---|---|
| 5.0 | 93.3 | 24.8 | 1.2 |
| 9.9 | 100 | 25.0 | 1.3 |
| 12.3 | 25.1 | 25.7 | 2 |
| 13.7 | 3.1 | 26.0 | 1.3 |
| 14.9 | 14.9 | 26.3 | 0.6 |
| 15.2 | 5.5 | 26.5 | 0.6 |
| 18.6 | 4.6 | 27.1 | 6.9 |
| 19.4 | 0.7 | 28.9 | 3.2 |
| 19.9 | 19.1 | 29.4 | 0.5 |
| 20.2 | 27.5 | 30.1 | 9.9 |
| 22.6 | 1.2 | 30.7 | 0.9 |
| 23.2 | 0.4 | 31.6 | 1.4 |
| 23.6 | 1 | 33.6 | 1.9 |
| 24.4 | 13.3 | 33.9 | 0.6 |

4. The crystal form B according to claim 3, wherein the X-ray powder diffraction pattern thereof is shown in Figure 5;
and/or, the differential scanning calorimetry thermogram thereof has an endothermic peak with onset at 178.7±3.0 °C;
and/or, the DSC thermogram thereof is shown in Figure 6;
and/or, the thermogravimetric analysis curve thereof has a weight loss of 1.03% occurred at 63.4±3.0 °C;
and/or, the TGA thermogram thereof is shown in Figure 7.

5. A crystal form C of the compound represented by formula (I), wherein, 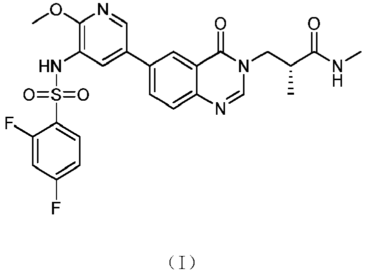
the X-ray powder diffraction pattern thereof has characteristic diffraction peaks at the following angles of 2θ: 4.9 ± 0.2°, 5.8 ± 0.2°, 6.8 ± 0.2°, 8.4 ± 0.2°, and 12.4±0.2°;
or, the X-ray powder diffraction pattern thereof has characteristic diffraction peaks at the following angles of 2θ: 4.9 ± 0.2°, 5.8 ± 0.2°, 6.8 ± 0.2°, 8.4 ± 0.2°, 10.8 ± 0.2°, 11.7 ± 0.2°, 12.4 ± 0.2°, 14.3 ± 0.2°, 17.0 ± 0.2°, 17.7 ± 0.2°, and 18.7 ± 0.2°.
or, in the X-ray powder diffraction pattern thereof, the angles of 2θ are shown in the following table:
| Angles of 2θ (°) | Relative intensity (%) | Angles of 2θ (°) | Relative intensity (%) |
|---|---|---|---|
| 4.2 | 9.8 | 14.3 | 22.7 |
| 4.9 | 43.5 | 14.7 | 8.3 |
| 5.1 | 8.8 | 14.9 | 7.7 |
| 5.8 | 38.2 | 15.3 | 8.1 |
| 6.2 | 9.3 | 15.9 | 17.3 |
| 6.8 | 27.6 | 17.0 | 31.9 |
| 7.4 | 8.1 | 17.2 | 9.5 |
| 7.5 | 14.7 | 17.7 | 21.3 |
| 8.4 | 31.4 | 18.7 | 25.4 |
| 9.6 | 16.4 | 19.3 | 14.4 |
| 10.3 | 4.3 | 19.7 | 6.2 |
| 10.8 | 19.3 | 20.5 | 7.2 |
| 11.4 | 28.4 | 21.8 | 12 |
| 11.7 | 31.6 | 22.8 | 14.1 |
| 12.0 | 23.4 | 23.5 | 7.4 |
| 12.4 | 100 | 23.9 | 6.7 |
| 13.2 | 12.3 | 24.1 | 13 |
| 13.5 | 5.4 | 25.0 | 3.6 |

6. The crystal form C according to claim 5, wherein the X-ray powder diffraction pattern thereof is shown in Figure 8.

7. A crystal form D of the compound represented by formula (I), wherein, 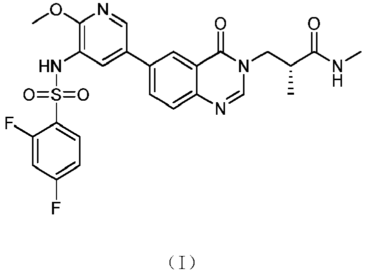
the X-ray powder diffraction pattern thereof has characteristic diffraction peaks at the following angles of 2θ: 5.1 ± 0.2°, 7.8 ± 0.2°, and 11.8±0.2°;
or, the X-ray powder diffraction pattern thereof has characteristic diffraction peaks at the following angles of 2θ: 5.1±0.2°, 6.5±0.2°, 7.8±0.2°, 11.8±0.2°, 15.4±0.2°, 16.5±0.2°, 17.4±0.2° and 23.8±0.2°;
the X-ray powder diffraction pattern thereof has characteristic diffraction peaks at the following angles of 2θ: 5.1 ± 0.2°, 6.5 ± 0.2°, 7.8 ± 0.2°, 11.8 ± 0.2°, 12.0 ± 0.2°, 15.4 ± 0.2°, 16.5 ± 0.2°, 17.4 ± 0.2°, 19.5 ± 0.2°, and 23.8 ± 0.2°.
or, in the X-ray powder diffraction pattern thereof, the angles of 2θ are shown in the following table:
| Angles of 2θ (°) | Relative intensity (%) | 2θ (°) | Relative intensity (%) |
|---|---|---|---|
| 5.1 | 100 | 22.3 | 8.7 |
| 6.5 | 16.3 | 23.8 | 23.1 |
| 7.3 | 8.3 | 24.0 | 13.1 |
| 7.8 | 95.4 | 24.6 | 4.8 |
| 11.8 | 71.9 | 25.3 | 5.9 |
| 12.0 | 44.3 | 26.3 | 16.7 |
| 12.8 | 13.5 | 26.6 | 14.3 |
| 14.1 | 10.1 | 27.0 | 8.4 |
| 15.4 | 35.5 | 28.1 | 16.1 |
| 16.5 | 50.3 | 28.5 | 8.1 |
| 17.4 | 19.8 | 30.0 | 7.3 |
| 17.9 | 4.4 | 30.6 | 3.7 |
| 18.6 | 11 | 31.5 | 4 |
| 19.5 | 41 | 32.4 | 4.2 |
| 20.2 | 4.4 | 33.6 | 3.4 |
| 20.8 | 4.4 | 35.8 | 3.1 |
| 21.4 | 4.5 | 38.0 | 3.5 |

8. The crystal form D according to claim 7, wherein the X-ray powder diffraction pattern thereof is shown in Figure 9.

9. A preparation method of the crystal form A of the compound represented by formula (I), the preparation method is method 1 or method 2,
method 1 comprises:
(1) adding a compound represented by formula (I) into a solvent to make it into a suspension or solution;
(2) putting the suspension or solution in a constant temperature mixer, shaking, then centrifuging, and drying to obtain the crystal form A of the compound represented by formula (I);
method 2 comprises:
(1) adding a compound represented by formula (I) into a solvent and heating to dissolve;
(2) cooling the solution until solid precipitates, stirring and filtering to obtain the crystal form A of the compound of formula (I).

10. The preparation method according to claim 9, wherein the solvent is selected from alcohol solvent and ester solvent;
and/or, the solvent is selected from ethanol, *n*-butanol, *tert*-butanol, isopropanol, ethyl formate and ethyl acetate.

11. A preparation method of the crystal form B of the compound represented by formula (I), comprising:
(1) adding a compound represented by formula (I) into a solvent to make it into a suspension or solution;
(2) putting the suspension or solution in a constant temperature mixer, shaking, then centrifuging, and drying to obtain the crystal form B of the compound represented by formula (I).

12. The preparation method according to claim 11, wherein the solvent is selected from methanol-water, ethanol-water and acetone-water;
and/or, the solvent is selected from methanol-water (2:1), ethanol-water (2:1), acetone-water (2:1), ethanol-water (1:3).

13. The preparation method according to any one of claims 9 to 12, wherein the stirring temperature is 25°C to 60°C;
and/or, the stirring time is 12 hours to 24 hours;
and/or, the weight-volume ratio of compound to solvent is 1 g: 7 to 10 mL.

14. A use of the crystal form according to any one of claims 1 to 8 in the manufacture of a medicament for treating PI3Kα inhibitor-related diseases.

15. The use according to claim 14, wherein, the PI3Kα inhibitor-related medicaments are medicaments for tumors.

16. A pharmaceutical composition comprising the crystal form according to at least one of claims 1-8 and a pharmaceutically acceptable excipient.

17. A use of the crystal form according to at least one of claims 1 to 8 or the pharmaceutical composition according to claim 16 in the preparation of PI3K inhibitors;
the PI3K inhibitor is preferably an inhibitor of one or more of PI3Kα, PI3Kβ, PI3Kδ and PI3Kγ, more preferably an inhibitor of PI3Kα.

18. A use of the crystal form according to at least one of claims 1 to 8 or the pharmaceutical composition according to claim 16 in the preparation of a medicament;
the medicament is preferably a medicament for treating tumors or a medicament for PI3K-related diseases;
the PI3K-related disease is preferably a tumor;
the PI3K is preferably one or more of PI3Kα, PI3Kβ, PI3Kδ and PI3Kγ, more preferably PI3Kα.

19. A method for treating tumors or PI3K-related diseases comprising administering a therapeutically effective amount of the crystal form according to at least one of claims 1 to 8 or the pharmaceutical composition according to claim 16 to a patient;
the PI3K-related disease is preferably a tumor;
the PI3K is preferably one or more of PI3Kα, PI3Kβ, PI3Kδ and PI3Kγ, more preferably PI3Kα.
